# EUROPEAN PATENT SPECIFICATION

(11) **EP 0 411 211 B1**
(45) Date of publication and mention of the grant of the patent: **20.10.1993**
(21) Application number: 89202040.5
(22) Date of filing: 04.08.1989
(51) Int. Cl.: A61K 7/18

(54) **Oral compositions**
Oral-Präparate
Compositions buccales

(43) Date of publication of application: 06.02.1991
(73) Proprietor: UNILEVER N.V., 3013 AL Rotterdam (NL); UNILEVER PLC, London EC4P 4BQ (GB)
(72) Inventor: Evans, Kenneth Arthur, Chalfont St. Peter Bucks, SL9 0 DX (GB); Riley, Paul Ian, Wirral Merseyside, L63 9AA (GB); Rossall, Brian, Wirral Merseyside (GB)
(74) Representative: van Gent, Jan Paulus

(56) References cited:
- EP-A- 0 251 146
- EP-A- 0 304 325
- EP-A- 0 328 407

## Description

This invention relates to oral compositions, more particularly to oral compositions comprising as an abrasive cleaning agent particles of alumina trihydrate (Al₂O₃3H₂O), sometimes referred to as aluminium trihydrate or aluminium hydroxide.

Alumina trihydrate is a well-known abrasive agent of oral compositions such as toothpastes. It is made by the Bayer process which produces a coarse grade of material which is then milled to a particle size appropriate for use in an oral composition.

In GB-A-1 277 585 (Unilever) it is disclosed that toothpastes containing milled alumina trihydrate can cause corrosion of aluminium toothpaste tubes and that sodium monofluorophosphate and other sources of monofluorophosphate ions are effective to inhibit this corrosion. Certain phosphate esters are also disclosed as being effective in GB-A-1 475 252 (Colgate-Palmolive). The presence of certain materials during the milling process as disclosed in GB-A-1 537 823 (British Aluminium Co. Ltd) and GB-A-1 544 537 (Colgate-Palmolive) is said to modify the surface of the milled alumina trihydrate particles and make them more suitable for inclusion in toothpastes contained in aluminium tubes.

It is nowadays common to include sodium monofluorophosphate in dentifrices as a source of fluoride to protect the teeth against dental caries.

We have determined that there is some interaction between sodium monofluorophosphate and an alumina trihydrate abrasive resulting in a loss of soluble fluoride on storage of a dentifrice, which is more marked at elevated temperatures. Thus, there may be some loss of anti-caries efficacy of such toothpastes.

It has now been discovered that the stability of monofluorophosphate in dentifrices containing an alumina trihydrate abrasive, and also containing zinc citrate, is surprisingly improved if instead of employing a milled alumina trihydrate there is used an unmilled precipitated alumina trihydrate of appropriate particle size. The stability of soluble zinc in the dentifrice is also improved. Zinc citrate is a know anti-calculus and anti-plague agent (see US-A-4 100 269 (Lever Brothers Co.)

Accordingly the invention relates to an oral composition comprising 20 to 70% by weight of an unmilled precipitated alumina trihydrate of average particle size 3 to 15 µm, preferably 5 to 10 µm, sodium monofluorophosphate in an amount of 0.1 to 2% by weight and zinc citrate in an amount of 0.02 to 5% by weight.

"EP-A-0 251 146 describes a toothpaste comprising sodium monofluorophosphate, zinc citrate and alumina trihydrate with a particle size of between 1 and 20 µm, especially about 10 µm.

EP-A-0 304 325 also describes a toothpaste comprising sodium monofluorophosphate, zinc citrate and alumina trihydrate.

Neither reference discloses, however, that the alumina trihydrate is of the unmilled, precipitated type. EP-A- 0 328 407 discloses toothpastes comprising sodium monofluorophosphate and unmilled precipitated alumina trihydrate, but there is no disclosure of the additional presence of a water-soluble zinc-compound"

The alumina abrasive used in oral compositions of this invention is made by a precipitation process which is a modification of that conventionally used to produce very fine alumina precipitates of average particle size 1-2 µm and below, in which process the precipitation conditions are varied so as to allow the growth of larger crystal aggregates. A suitable method to prepare the aluminas is described by J. Scott in a paper, "The effect of Seed and Temperature on the Particle Size of Bayer Hydrate", presented at the International Symposium on the Extractive Metallurgy of Aluminium, New York February 1962.

As indicated above, it has been found that the unmilled precipitated alumina abrasive has improved compatibility with soluble zinc and therefore in accordance with a further aspect of the invention there is provided an oral composition comprising in combination an unmilled precipitated alumina trihydrate abrasive and a water-soluble zinc compound. It is well known to include water-soluble zinc compounds in oral compositions. Many are disclosed in US-A-4 100 269 referred to above. Others are disclosed in US-A-4 022 880, US-A-4 144 323, US-A-4 656 031 and US-A-4 160 821. The amount of the zinc compound may be from about 0.02% to about 5% by weight of the oral composition.

Oral composition of the invention will also contain other conventional ingredients. Those in the form of a dentifrice cream or gel will usually contain an humectant liquid, a surface-active agent, a binder or thickener and flavour. Other minor ingredients may also be present, such as up to 1% by weight of 2',4,4'-trichloro-2-hydroxy-diphenylether as antimicrobial agent.

In GB-A-2 009 596 (Unilever) there is described a toothpaste containing MFP and alumina trihydrate abrasive but not containing zinc ion-producing compounds, wherein the abrasive consists of a mixture of an alumina trihydrate having an average particle size of from 5 to 13 µm and an alumina trihydrate having an average particle size of less than 1 µm, the weight ratio of the two alumina trihydrates being from 30:70 to 70:30. The sub-µm alumina trihydrate described has an average particle size of 0.2 to 0.8 µm, particularly about 0.5 µm. It is produced commercially as a fine precipitate and not by grinding larger particles.

The following experiments illustrate the invention. Percentages are by weight.

Toothpastes were made having the following composition.

| Ingredient | % |
|---|---|
| Alumina trihydrate | 50.0 |
| Sorbitol syrup (70% solution) | 27.0 |
| Sodium lauryl sulphate | 1.875 |
| Sodium dodecylbenzene sulphonate | 0.6 |
| Sodium carboxymethylcellulose | 0.8 |
| Zinc citrate trihydrate | 0.5 |
| Sodium monofluorophosphate | 0.85 |
| Flavour | 1.2 |
| Sodium saccharin | 0.2 |
| Formalin | 0.04 |
| Colour | 0.006 |
| Water | to 100.0 |

A number of toothpastes were formulated using the different grades of alumina trihydrate mentioned below:
1. Milled alumina trihydrate of average particle size 6-8 µm (available commercially from BA Chemicals as AF 280).
2. Milled alumina trihydrate of average particle size about 10 µm (available commercially from BA Chemicals as AF 240).
3. A precipitated (not milled) alumina trihydrate of average particle size about 6 µm.

The respective toothpastes were stored at 37°C for 6 months. The amount of water-soluble fluoride available after storage expressed as a percentage of that available immediately after manufacture is indicated in the Table below. The percentage of soluble zinc is also given in the Table.

| Abrasive present | % F | % Zn |
|---|---|---|
| Milled alumina trihydrate, aps* 6-8 µm | 81 | 48 |
| Milled alumina trihydrate, aps about 10 µm | 92 | 37 |
| Precipitated alumina trihydrate, aps about 6 µm | 99 | 54 |

| | | |
|---|---|---|
| * average particle size | | |

The results show the superior properties of the toothpaste according to this invention.

## Claims

1. An oral composition comprising 0.1-2 % by weight of sodium monofluorophosphate, 0.02-5 % by weight of a water-soluble zinc compound and from 20-70 % by weight of an alumina trihydrate with an average particle size of between 3 and 15 µm, characterised in that the alumina trihydrate is an unmilled, precipitated alumina trihydrate.

2. A composition according to claim 1, wherein the zinc compound is zinc citrate.

3. A composition according to claim 1, wherein the alumina trihydrate has an average particle size of between 5 and 10 µm.

## Patentansprüche

1. Orale Zusammensetzung umfassend 0,1-2 Gew.-% Natriummonofluorphosphat, 0,02-5 Gew.-% einer wasserlöslichen Zinkverbindung und 20-70 Gew.-% eines Aluminiumoxidtrihydrats mit einer durchschnittlichen Teilchengröße von zwischen 3 und 15 µm, dadurch gekennzeichnet, daß das Aluminiumoxidtrihydrat ein ungemahlenes, ausgefälltes Aluminiumoxidtrihydrat ist.

2. Zusammensetzung gemäß Anspruch 1, worin die Zinkverbindung Zinkzitrat ist.

3. Zusammensetzung gemäß Anspruch 1, worin das Aluminiumoxidtrihydrat eine durchschnittliche Teilchengröße von zwischen 5 und 10 µm hat.

## Revendications

1. Composition buccale comprenant de 0,1 à 2% en poids de monofluorophosphate de sodium, de 0,02 à 5% en poids d'un composé de zinc hydrosoluble et de 20 à 70% en poids d'une alumine trihydratée ayant une granulométrie moyenne de 3 à 15 µm, caractérisée en ce que l'alumine trihydratée est une alumine trihydratée précipitée et non broyée.

2. Composition selon la revendication 1, dans laquelle le composé de zinc est le citrate de zinc.

3. Composition selon la revendication 1, dans laquelle la granulométrie moyenne de l'alumine trihydratée est de 5 à 10 µm.
